# EUROPEAN PATENT APPLICATION

(11) **EP 3 912 645 A1**
(43) Date of publication of application: **24.11.2021**
(21) Application number: 21173685.5
(22) Date of filing: 12.05.2021
(51) Int. Cl.: A61L 2/00, A61L 2/18, A61L 2/22

(54) **APPARATUS FOR SANITIZING PROTECTION DEVICES**

(30) Priority: 18.05.2020 IT 202000002599 U
(71) Applicant: C.M.S. S.p.A., 41054 Marano Sul Panaro, Modena (IT)
(72) Inventor: MAGGI, Marco, 41058 VIGNOLA, Province of Modena (IT); DEGLI ESPOSTI, Vainer, 41059 ZOCCA, Province of Modena (IT)
(74) Representative: Mola, Edoardo

(57) **Abstract**

The present invention relates to an apparatus for sanitizing individual protection devices, such as gloves and masks. The device comprises: a) a main body; b) a closable opening designed to allow the introduction of these protection devices to be sanitized inside the main body; c) a photocell designed to automatically allow access from the outside to the opening; d) a collection device, located inside the main body beneath the opening, designed to collect the protection devices once inserted into the main body; and e) a sanitizing system designed to spray the protection devices with a partially inerting solution inside the main body.

In this way, when the user approaches the apparatus to throw the protection devices to be sanitized inside it, the photocell automatically activates the opening, consequently creating a passage that allows these devices to be inserted inside the apparatus for sanitizing without the user coming into contact with any element of the apparatus.

In addition, these individual protection devices, once inside the apparatus, fall by gravity and are stored in the appropriate collection device

## Description

### TECHNICAL FIELD

The present invention relates to the field of apparatus for sanitizing devices; in particular, it relates to the field of apparatus for sanitizing individual protection devices, such as masks and gloves after use

### BACKGROUND OF THE INVENTION.

These days, when the world is living in a state of emergency due to the coronavirus, a massive use of individual protection device, such as masks and gloves, is made to prevent contagion.

These individual protection devices are usually disposable, or in any case they can only be reused for a short period of time; at the end of this time, they must be disposed of after undergoing an inertization treatment to reduce the possibility that the virus, possibly nested in these devices, being transmitted to other people.

Therefore, there is a need to sanitize these individual protection devices once used.

Therefore, the applicant of this application has found a need to create an apparatus for sanitizing individual protection devices

### SUMMARY OF THE INVENTION

The present invention refers to an apparatus as claimed in claim 1.

The present invention arises from the general consideration according to which the technical problem highlighted above can be solved effectively and reliably by an apparatus for sanitizing individual protection devices, such as gloves and masks, an apparatus comprising: a) a main body ; b) a resealable opening designed to allow the insertion inside the main body of the individual protection devices to be sanitized; c) a photocell designed to automatically allow access from the outside to said closable opening; d) a collection device, located within the main body beneath said closable opening, designed to collect said individual protection devices once placed within said main body; and e) a sanitizing system designed to spray said individual protection devices with a partially inerting solution within said main body.

In this way, to throw the individual protection devices to be sanitized inside, the photocell automatically activates the opening, thus creating a passage that allows these devices to be inserted inside of the apparatus for partial sanitization without the user coming into contact with any element of the apparatus.

In addition, these individual protection devices, once inside the apparatus, fall by gravity and are collected in the appropriate collection device.

According to a preferred embodiment, said main body is substantially parallelepipedal in shape, with vertical development.

According to a preferred embodiment, said device further comprises a sliding door adapted to be activated by said photocell to create said closable opening and allow access thereto from the outside.

In this way, in conditions where there is no protection device for sanitisation inside the apparatus main body, the door is in a position such that said closable opening is not made and therefore cannot be used in any way for the passage of objects. Conversely, when the photocell detects the presence of a user in the immediate proximity of the device, this door slides on special guides making said opening visible and therefore usable for the intended purposes.

According to a preferred embodiment, said collection device is for example a bag.

According to another alternative preferred embodiment, said collection device is a container, typically made of plastic or cardboard, substantially parallelepipedal in shape, such as those of a commercial type provided by municipalities for the collection of material to be disposed of.

According to a preferred embodiment, said apparatus also contains within in a support frame in which said bag can be fixed.

In this way, said bag can be hung vertically on said frame, with the upper edge of the bag anchored in a removable way to said frame, in order to remain open to facilitate the fall into the protection devices to be sanitized.

According to a preferred embodiment, said sanitizing system comprises a container, such as a tank or cistern, capable of containing said partially inertizing solution within it.

According to a preferred embodiment, said container, type tank or cistern, is positioned at the bottom of the main body of the apparatus.

In this way, the container rests directly on the base of the main body, without the needing any additional support to support itself.

According to a preferred embodiment, said sanitizing system comprises at least one nozzle adapted to spray said partially inerting solution towards said individual protection devices to be sanitized.

In this way it is possible to direct the solution jet appropriately towards said protection devices.

According to a preferred embodiment, said sanitizing system further comprises at least one pump able to allow said solution contained in said container to reach said at least one nozzle to be supplied to said individual protection devices to be sanitized.

In this way, the pump allows the withdrawal of a certain quantity of said solution from the respective container and the subsequent delivery of said solution towards said nozzles, even if said at least one nozzle is positioned vertically higher than the container where said solution is located.

According to a preferred embodiment, at least one of said at least one nozzle is arranged along the fall path of said individual protection devices from said opening towards said collection device.

In this way, the individual protection devices to be sanitized are hit by the partially inerting solution as they fall towards the collection device.

According to another alternative preferred embodiment, at least one of said at least one nozzle is arranged in correspondence with said collection device.

In this way, the individual protection devices to be sanitized are hit by the partially inerting solution when they are placed inside the collection device.

According to yet another further preferred embodiment, at least one of said at least one nozzle is arranged along the fall path of said individual protection devices from said closable opening towards said collection device, and at least another of said at least one nozzle it is arranged in correspondence with said collection device.

In this way, the individual protection devices to be sanitized are affected by the partially inerting solution both during their fall towards the collection device and when they are placed inside the collection device.

According to a preferred embodiment, said partially inerting solution comprises a mixture of water and alcohol.

According to a preferred embodiment, said solution comprises from 20 to 40% of water and from 60 to 80% of alcohol.

According to a preferred embodiment, the alcohol content is about 70 °.

According to a preferred embodiment, the apparatus of the present invention further comprises an inclined plane placed within said main body beneath said closable opening.

In this way, the falling of said individual protection devices towards said collection device is facilitated.

According to a preferred embodiment, said inclined plane is formed by a sheet which acts as a chute.

According to another preferred embodiment, said inclined plane is formed by a series of consecutive rollers placed next to each other.

In this way, the falling of said individual protection devices towards said collection device is further facilitated.

According to a preferred embodiment, the apparatus of the present invention also comprises a further opening, positioned in the lower part of said main body, suitable for allowing the withdrawal of said collection device.

In this way, when the user believes that the collection device contains an adequate number of sanitized protection devices, ready for disposal, it is possible, through said further opening, to withdraw said collection device from inside the main body of the device.

The collection device, treated with the necessary precautions, is then ready to be transported to an area suitable for the disposal of at least partially sanitised protection devices.

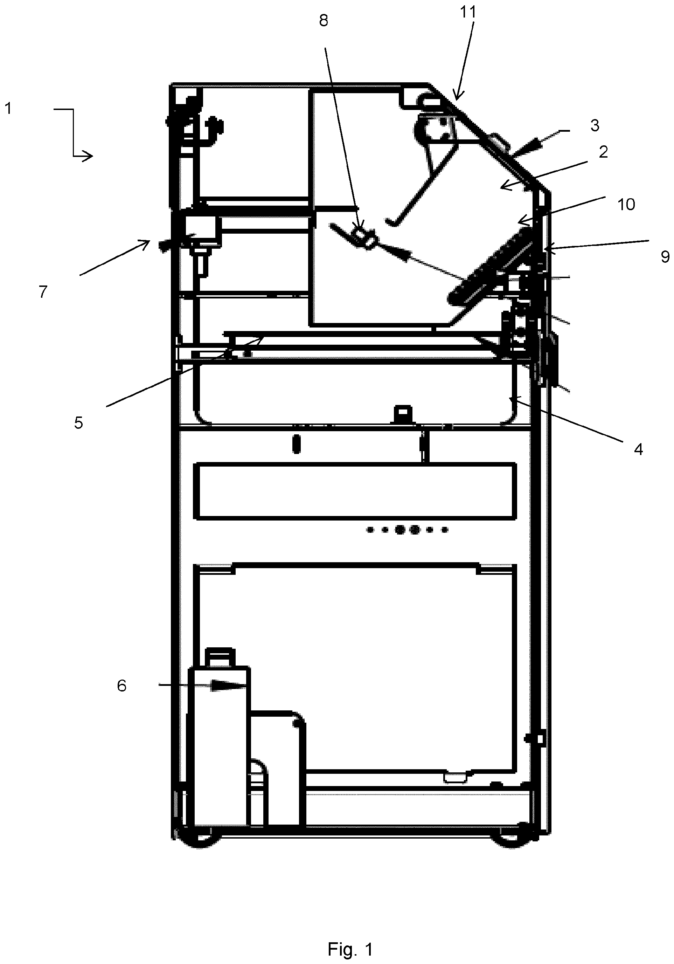

Further features and advantages of the present invention will be better highlighted by examining the following detailed description of a preferred but non-exclusive embodiment, illustrated by way of non-limiting example, with the support of the attached drawing, wherein Figure 1 is a view front of a first embodiment of the present invention.

### DETAILED DESCRIPTION.

The following detailed description refers to an embodiment of an apparatus of the present invention shown in Fig.1.

Fig.1 shows an apparatus for sanitizing individual protection devices, such as gloves and masks. The device comprises a main body 1 with a substantially parallelepipedal shape, with vertical development.

A photocell 11 is capable to detect the presence of a user in proximity of the apparatus and, consequently, automatically trigger the sliding of a sliding door 3 until it forms an opening 2, located laterally in the upper part of the main body 1, through which the user can introduce the protection devices to be sanitized inside the main body 1 of the apparatus. Once the introduction of the protection devices inside apparatus has been completed, the user moves away from the apparatus itself; consequently, the photocell 11 no longer detects the presence of the user at the apparatus and the door 3 automatically slides backwards until the opening 2 is no longer visible from the outside.

Inside the main body 1, beneath the opening 2, there is a collection device 4, such as a bag 4, capable of connecting by gravity the protection devices to be sanitized, once they have been inserted into the inside the main body 1 through the opening 2. The upper part of the bag 4 is fixed to a support frame 5, so that the bag 4 hangs vertically on the frame 5. The bag 4 thus remains open to facilitate the falling of the protection devices to be sanitized in the bag 4.

Inside the main body 1, beneath the opening 2, there is an inclined plane 9 designed to facilitate the fall of the protection devices to be sanitized towards the bag 4; the inclined plane 9 is formed by a series of rollers 10, placed next to each other.

The device also comprises inside the main body 1 a sanitization system suitable for spraying the individual protection devices once inserted into the main body 1 of the apparatus.

The sanitizing system comprises a container 6 suitable for containing a partially inerting solution, a pump 7 suitable for picking a certain quantity of this solution from the container 6 and sending it to one or more nozzles 8 to distribute the solution to the individual protection devices to be sanitized.

The container 6 is a tank or cistern, designed to contain the partially inertizing solution and is positioned on the bottom of the main body 1 of the apparatus, so that no additional support is required to support the container 6.

At least a nozzle 8 is arranged along the falling path of the protection devices to be sanitized, from opening 2 towards bag 4. At least another nozzle can be arranged in correspondence with the bag 4.

The partially inerting solution comprises a mixture composed of about 30% of water and about 70% of alcohol having an alcohol content of 70 °. This type of solution allows to reduce the infectious risk.

The apparatus also comprises a further opening, positioned in the lower part of the main body 1, designed to allow a user to be able to access the internal part of the main body 1 to free the upper part of the bag 4 from the frame 5 and then, subsequently, remove the bag 4 itself containing the sanitized protection devices. In this way, when the user considers that the bag 4 contains an adequate number of sanitized protection devices, ready for disposal,the bag 4 is removed and replaced with a new bag 4.

The bag 4, treated with the necessary precautions, is then ready to be transported to a suitable area for the disposal of the protection devices that have been at least partially sanitized.

The apparatus of the present invention can be used both hospital and corporate environment.

Of course, many modifications and variations of the preferred embodiments described will be clear to those people skilled in art while remaining within the scope of the invention.

Therefore, the present invention is not limited to the preferred embodiment described, illustrated only as a non-limiting example, but is defined by the following claims.

## Claims

1. Apparatus for sanitizing individual protection devices, such as gloves and masks, comprising: a) a main body (1); b) a resealable opening (2) suitable to allow the introduction inside said main body (1) of said individual protection devices to be sanitized; c) a photocell (11) suitable to automatically provide access from the outside to said closable opening (2); d) a collection device (4), placed inside said main body (1) below said closable opening (2), suitable to collect said individual protection devices once inserted inside said main body (1); and e) a sanitization system suitable for spraying said individual protection devices with a partially inertizing solution inside said main body (1).

2. Apparatus according to claim 1 also comprising a sliding door (3) suitable to be activated by said photocell (11) to create said closable opening (2) and provide access to it from the outside.

3. Apparatus according to any of the preceding claims, wherein said sanitizing system comprises a container (6) suitable for containing said solution.

4. Apparatus according to any of the preceding claims, wherein said sanitizing system comprises at least one nozzle (8) adapted to spray said solution towards said individual protection devices to be sanitized.

5. Apparatus according to any of the preceding claims 3 or 4, wherein said sanitizing system further comprises at least one pump (7) suitabe to allow said solution contained in said container (6) to reach said at least one nozzle (8) to be delivered to said individual protection devices to be sanitized.

6. Apparatus according to any of the preceding claims, wherein said partially inertizing solution comprises a mixture of water and alcohol.

7. Apparatus according to claim 6 wherein said partially inertizing solution comprises from 20 to 40% of water and from 60 to 80% of alcohol.

8. Apparatus according to any of the preceding claims, wherein at least one of said at least one nozzle (8) is arranged along the path of gravity fall of said individual protection devices from said closable opening (2) towards said collection device (6).

9. Apparatus according to any of the preceding claims, in which at least one of said at least one nozzle (8) is arranged in correspondence with said collection device (6).

10. Apparatus according to any of the preceding claims which further comprises an inclined plane (9) placed inside said main body (1) below said closable opening (2) suitable to facilitate the gravity fall of said individual protection devices towards said collection device (6).
